(19) Europäisches Patentamt
European Patent Office
Office européen des brevets

(11) EP 4 692 123 A1

(12) **EUROPEAN PATENT APPLICATION**
published in accordance with Art. 153(4) EPC

(43) Date of publication:
**11.02.2026 Bulletin 2026/07**

(21) Application number: **24785143.9**

(22) Date of filing: **01.04.2024**

(51) International Patent Classification (IPC):
***C07K 16/28*** (2006.01) ***A61K 47/68*** (2017.01)
***A61P 35/00*** (2006.01) ***A61K 39/00*** (2006.01)

(52) Cooperative Patent Classification (CPC):
**A61K 39/00; A61K 47/68; A61P 35/00; C07K 16/28**

(86) International application number:
**PCT/KR2024/004194**

(87) International publication number:
**WO 2024/210437 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.04.2023 KR 20230044305**

(71) Applicant: **Genome & Company, Inc.**
**Suwon-si, Gyeonggi-do, 16229 (KR)**

(72) Inventors:
- **CHA, Mi Young**
  **Seongnam-si, Gyeonggi-do 13486 (KR)**
- **KIM, Hyunuk**
  **Seongnam-si, Gyeonggi-do 13486 (KR)**
- **BYUN, Seungmin**
  **Seongnam-si, Gyeonggi-do 13486 (KR)**
- **HA, Youngeun**
  **Seongnam-si, Gyeonggi-do 13486 (KR)**
- **KIM, Mira**
  **Seongnam-si, Gyeonggi-do 13486 (KR)**
- **YU, Hyunkyung**
  **Seongnam-si, Gyeonggi-do 13486 (KR)**
- **MOON, Soo Jung**
  **Seongnam-si, Gyeonggi-do 13486 (KR)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) ANTI-BCAM ANTIBODY AND ANTIBODY-DRUG CONJUGATE

(57) The present invention provides an antibody or an antigen-binding fragment thereof that binds to basal cell adhesion molecule (BCAM), an antibody-drug conjugate comprising the antibody or the antigen-binding fragment thereof, a nucleic acid encoding the antibody or the antigen-binding fragment thereof, and a method for obtaining such an antibody or an antigen-binding fragment thereof and the antibody-drug conjugate. Furthermore, the present invention relates to the use of the antibody or the antigen-binding fragment thereof and the antibody-drug conjugate for the prevention, amelioration, or treatment of a disease associated with BCAM function or expression (for example, cancer).

**[Figure 1]**

| Region | Sequence |
|---|---|
| Ab-I VH | EVQLVESGGGLVKPGGSLRLSCAASGFTFSSYSMNWVRQAPGKGLEWVS SISSSSSYIYYADSVKGRFTISRDNAKNSLYLQMNSLRAEDTAVYYC ARIIGGWLGDYWGQGTLVTVSS |
| Region | Sequence |
| Ab-I VL | NIQMTQSPSSVSASVGDRVTITCRASQGISSWLAWYQQKPGKAPKLLIY AASSLRSGVPSRFSGSGSGTDFTLTISSLQPEDFATYYCQQANSFPPPT FGGGTKVEIK |



Processed by Luminess, 75001 PARIS (FR)

## Description

## [Technical Field]

### Cross-reference to related application

This application claims priority to Korean Patent Application No. 10-2023-0044305, filed on April 4, 2023, entitled "ANTI-BCAM ANTIBODY AND ANTIBODY-DRUG CONJUGATE", the disclosure of which is incorporated herein by reference in its entirety.

### Technical field

The present invention relates to an anti-BCAM antibody or an antigen-binding fragment thereof that binds to a basal cell adhesion molecule (BCAM) protein, an antibody-drug conjugate comprising the antibody or the antigen-binding fragment thereof, a pharmaceutical composition comprising the antibody, the antigen-binding fragment thereof or the antibody-drug conjugate, a method for producing the antibody, the antigen-binding fragment thereof or the antibody-drug conjugate and a use thereof, a polynucleotide encoding the antibody or the antigen-binding fragment thereof, a vector comprising the polynucleotide, and an isolated host cell for recombinantly producing the antibody or the antigen-binding fragment thereof. The antibody, the antigen-binding fragment thereof or the antibody-drug conjugate of the present invention may be used for the prevention, amelioration, or treatment of diseases associated with BCAM function or expression, such as cancer.

## [Background]

BCAM is a transmembrane protein of the immunoglobulin superfamily, also known as Lu (Lutheran blood group glycoprotein) or CD239, and Lu and BCAM are referred to interchangeably and are also called Lu/BCAM. Lu was initially studied as an antigen of the Lutheran blood group system, and BCAM was identified as an up-regulated antigen in ovarian cancer. Lu and BCAM have the same extracellular domain but differ in their cytoplasmic tails. Specifically, BCAM lacks the 40 C-terminal amino acids present in the Lu cytoplasmic tail. Furthermore, the Lu-specific cytoplasmic region contains an SH3-binding motif, a dileucine motif, and potential phosphorylation sites. However, the common region of the Lu and BCAM cytoplasmic tails includes a spectrin-binding motif. Due to this structural overlap between BCAM and Lu, it is known to be difficult to distinguish between Lu and BCAM in actual tissues.

The extracellular domain of BCAM comprises one V-set, one C1-set, and three I-set domains (V-C1-I-I-I). BCAM specifically binds to laminin $\alpha$5, a major component of the basement membrane. Laminin $\alpha$5 associates with $\beta$ and y chains to form a heterotrimer, which is found in many basement membranes of both normal and diseased tissues. Furthermore, BCAM promotes the migration of lung cancer cells on laminin-511 (LM-511), which consists of $\alpha$5, $\beta$1, and $\gamma$1 chains, and the migration of tumor cells on LM-511 is inhibited in the presence of a function-inhibiting antibody against BCAM.

In addition to ovarian cancer, overexpression of BCAM has also been observed in breast cancer, prostate cancer, skin cancer, hepatocellular carcinoma, and KRAS-mediated Colorectal Cancer (CRC), and accordingly, BCAM has been proposed as a useful antigen for antibody drugs and their use in cancer diagnosis.

## [Prior art document]

**Non-patent literature** - Yamato Kikkawa et al., Scientific Report, 2018 Apr 26;8(1):6612

## [Detailed Description of the Invention]

## [Problem to be Solved]

An object of the present invention is to provide an anti-BCAM antibody or an antigen-binding fragment thereof that binds to BCAM protein with high affinity, or an antibody-drug conjugate comprising the anti-BCAM antibody, and methods for producing the same.

Another object of the present invention is to provide a use of an anti-BCAM antibody or an antigen-binding fragment thereof, or an antibody-drug conjugate comprising the anti-BCAM antibody for the prevention, amelioration, treatment, or diagnosis of a disease associated with BCAM function or expression (e.g., cancer).

**[Means for Solving the Problem]**

[1] The present invention provides an anti-BCAM antibody or an antigen-binding fragment thereof that binds to a basal cell adhesion molecule (BCAM) protein, wherein the anti-BCAM antibody comprises a heavy chain CDR1 comprising the amino acid sequence of SEQ ID NO: 3; a heavy chain CDR2 comprising the amino acid sequence of SEQ ID NO: 4; a heavy chain CDR3 comprising the amino acid sequence of SEQ ID NO: 5; a light chain CDR1 comprising the amino acid sequence of SEQ ID NO: 6; a light chain CDR2 comprising the amino acid sequence of SEQ ID NO: 7; and a light chain CDR3 comprising the amino acid sequence of SEQ ID NO: 8.

[2] In accordance with [1], the present invention provides an anti-BCAM antibody or an antigen-binding fragment thereof, comprising a heavy chain variable region consisting of the amino acid sequence of SEQ ID NO: 1 and a light chain variable region consisting of the amino acid sequence of SEQ ID NO: 2.

[3] In accordance with [1] or [2], the antibody or antigen-binding fragment thereof is an anti-BCAM antibody or an antigen-binding fragment thereof of an IgG isotype.

[4] In accordance with any one of [1] to [3], the antibody or antigen-binding fragment thereof is a multi-specific antibody or an antigen-binding fragment thereof.

[5] In accordance with any one of [1] to [4], the antibody or antigen-binding fragment thereof is an anti-BCAM antibody or an antigen-binding fragment thereof that is a humanized antibody, a chimeric antibody, a CDR-grafted antibody, or a recombinant human antibody.

[6] In accordance with any one of [1] to [5], the antigen-binding fragment is Fab, Fab', Fab'-SH, Fv, a single-chain antibody scFv, an $F(ab')_2$ fragment, a light chain variable region (VL), a heavy chain variable region (VH), a diabody, a triabody, a tetrabody, a minibody, IgG delta $CH_2$, scFv-Fc, $(scFv)_2$-Fc, a Fynomer, a dual-affinity re-targeting (DART) protein, an anticalin, an $FN_3$ monobody, a DARPin, an Affibody, an Affilin, an Affimer, an Affitin, an Alphabody, an Avimer, Im7, VLR, VNAR, a Trimab, a CrossMab, a TRIDENT, a nanobody, a binanobody, or a di-sdFv.

[7] In accordance with any one of [1] to [6], the antibody or antigen-binding fragment thereof binds to human BCAM protein with a binding dissociation equilibrium constant ($K_D$) of 1 x $10^{-6}$ M or less, 1 x $10^{-7}$ M or less, 1 x $10^{-8}$ M or less, 1 x $10^{-9}$ M or less, 1 x $10^{-10}$ M or less, or 1 x $10^{-11}$ M or less.

[8] The present invention provides an antibody-drug conjugate comprising the anti-BCAM antibody or antigen-binding fragment thereof according to any one of [1] to [7].

[9] The present invention provides a pharmaceutical composition for the prevention, amelioration, or treatment of a disease associated with BCAM function or expression, comprising: the anti-BCAM antibody or antigen-binding fragment thereof according to any one of [1] to [7] or the antibody-drug conjugate of [8]; and a pharmaceutically acceptable carrier.

[10] In accordance with [9], the disease associated with BCAM function or expression is cancer.

[11] In accordance with [9] or [10], the pharmaceutical composition may further comprise an additional anticancer agent.

[12] In accordance with [11], the additional anticancer agent may be administered simultaneously with the antibody, antigen-binding fragment thereof or the antibody-drug conjugate in a single formulation, or simultaneously or sequentially in separate formulations.

[13] The present invention provides a nucleic acid (nucleotide) encoding the anti-BCAM antibody or antigen-binding fragment thereof according to any one of [1] to [7].

[14] The present invention provides a recombinant expression vector comprising the nucleic acid of [13].

[15] The present invention provides an isolated host cell for recombinantly producing the anti-BCAM antibody or

antigen-binding fragment thereof according to any one of [1] to [7].

[16] The present invention provides a method for producing the antibody or an antigen-binding fragment thereof, the method comprising: culturing the host cell of [15] under conditions that allow for the production of the antibody or antigen-binding fragment thereof, and isolating the antibody or antigen-binding fragment thereof from the host cell or culture medium.

[17] The present invention provides the anti-BCAM antibody or antigen-binding fragment thereof according to any one of [1] to [7] for use in the detection, diagnosis, and/or treatment of a disease associated with BCAM function or expression.

**[Effects of the Invention]**

The anti-BCAM antibody, an antigen-binding fragment thereof, or the antibody-drug conjugate comprising the antibody or the antigen-binding fragment thereof of the present invention binds to human BCAM protein with high affinity, and the anti-BCAM antibody has the effect of being internalized and accumulated in cancer cells expressing the BCAM protein. Therefore, the anti-BCAM antibody, the antigen-binding fragment thereof or the antibody-drug conjugate of the present invention can be usefully employed for the prevention, amelioration, or treatment of diseases associated with BCAM function or expression.

**[Brief Description of the Drawings]**

FIG. 1 shows the amino acid sequences of the heavy chain variable region and the light chain variable region of the Ab-I antibody, an example of the anti-BCAM antibody of the present invention. The underlined sequences indicate the CDR sequences of each region.

FIG. 2 shows the results of an ELISA analysis of the binding ability of the Ab-I antibody, an example of the anti-BCAM antibody of the present invention, to BCAM.

FIG. 3 shows the results of a FACS analysis of the binding ability of the Ab-I antibody, an example of the anti-BCAM antibody of the present invention, to BCAM; the values for PBS and human IgG are shown overlapping.

FIG. 4 shows the results of an Octet analysis of the binding ability of the Ab-I antibody, an example of the anti-BCAM antibody of the present invention, to BCAM.

FIG. 5 shows images of internalization degree of the Ab-I antibody, an example of the anti-BCAM antibody of the present invention, in the human cancer cell line SK-BR3, observed by confocal laser scanning microscopy.

FIG. 6 shows the results of an ELISA analysis of the binding ability of Ab-II, an example of the anti-BCAM antibody-drug conjugate of the present invention, to BCAM.

FIG. 7 shows the results of an analysis of the *in vitro* anticancer effect of Ab-II, an example of the anti-BCAM antibody-drug conjugate of the present invention, on the human cancer cell lines SK-BR3, OVCAR3, and DU145.

**[Best mode for the Invention]**

**Definitions**

The term "BCAM" as used herein refers to basal cell adhesion molecule, a surface glycoprotein that acts as a receptor for the extracellular matrix protein, laminin. "BCAM" is also known as Lutheran blood group glycoprotein (Lu) or CD239, and as used herein, "BCAM", "Lu", "Lu/BCAM", and "CD239" refer to the same protein and may be used interchangeably.

The term "antibody" as used herein refers to an immunoglobulin molecule that can specifically bind to a target, such as a carbohydrate, polynucleotide, lipid, polypeptide, protein, etc., via at least one antigen-recognition site located within the variable region of the immunoglobulin molecule. As used herein, the term "antibody" is used in its broadest sense and broadly includes intact polyclonal or monoclonal antibodies, as well as dimers, multimers, multi-specific antibodies

(e.g., bispecific antibodies), antigen-binding fragments thereof, antibody fragments, and fusion proteins comprising any other modified arrangements of immunoglobulin molecules containing antigen-recognition sites (e.g., variable regions), synthetic antibodies (e.g., "antibody mimetics"), "FynomAb", and the like.

There are five types of antibodies: Immunoglobulin (Ig) M, IgD, IgG, IgA, and IgE, each containing a heavy chain made from the heavy chain constant region genes $\mu$, $\delta$, $\gamma$, $\alpha$, and $\varepsilon$, respectively. The light and heavy chains of an antibody are divided into a variable region where the amino acid sequence differs among antibodies, and a constant region where the amino acid sequence is the same. The heavy chain constant region contains $CH_1$, H (hinge), $CH_2$, and $CH_3$ domains. Each domain is composed of two $\beta$-sheets, and an intramolecular disulfide bond connects them.

The term "antibody variable region" as used herein refers to the light chain and heavy chain portions of an antibody molecule that include the amino acid sequences of the CDRs and framework regions (FRs).

The term "complementarity-determining region (CDR)" as used herein refers to the amino acid residues of the antibody variable region that are necessary for antigen binding. Each variable region typically has three CDRs, identified as CDR1, CDR2, and CDR3.

The term "multi-specific antibody" as used herein refers to an antibody that has binding specificity for at least two different sites.

The terms "humanized antibody" and "CDR-grafted antibody" as used herein refer to an antibody in which one or more CDR sequences derived from a non-human species, such as another mammalian species, are inserted into the framework sequence from a human immunoglobulin molecule. The framework sequence may be further modified, for example, through mutation. Human Ig sequences can be referenced, for example, from the NCBI Database (Entrez Gene). By using appropriate sequences, the immunogenicity of the antibody can be reduced, or its binding properties, affinity, on-rate, off-rate, avidity, specificity, half-life, or any other suitable characteristic can be reduced, enhanced, or altered.

The term "chimeric antibody" as used herein refers to an antibody in which the variable region sequence is derived from one species and the constant region sequence is derived from another species, for example, an antibody in which the variable region sequence is derived from a mouse antibody and the constant region sequence is derived from a human antibody. Methods for producing chimeric antibodies are well known in the art. For example, Morrison, Science 229:1202 (1985) can be consulted, which is incorporated herein by reference in its entirety.

The term "human antibody" as used herein refers to an antibody that includes a variable region in which both the framework and CDR regions are derived from human immunoglobulin sequences. The constant region of the antibody is also derived from human immunoglobulin sequences.

The term "anti-BCAM antibody" as used herein includes not only monovalent antibodies with single specificity but also multi-specific antibodies comprising at least two arms (e.g., a first arm bound to BCAM and a second arm bound to a second (target) antigen).

The term "antigen-binding fragment" or "antibody fragment" as used herein typically refers to a fragment that comprises at least a portion of the antigen-binding domain (e.g., one or more CDRs) or the variable region of a parent antibody. An antibody fragment retains at least a portion of the binding specificity of the parent antibody. Examples of antigen-binding fragments that can be used herein include, but are not limited to, Fab, Fab', Fab'-SH, Fv, a single-chain antibody scFv, an $F(ab')_2$ fragment, a light chain variable region (VL), a heavy chain variable region (VH), a diabody, a triabody, a tetrabody, a minibody, IgG delta $CH_2$, scFv-Fc, $(scFv)_2$-Fc, a Fynomer, a dual-affinity re-targeting protein, an anticalin, an $FN_3$ monobody, a DARPin, an Affibody, an Affilin, an Affimer, an Affitin, an Alphabody, an Avimer, Im7, VLR, VNAR, a Trimab, a CrossMab, a TRIDENT, a nanobody, a binanobody, or a di-(di)-sdFv.

The light chain variable region (VL) refers to the variable light chain and is also called the VL domain.

The VH refers to the variable heavy chain and is also called the VH domain.

The Fab refers to a monovalent fragment consisting of VL, VH, CL, and $CH_1$ domains.

The Fab' differs from the Fab fragment in that a few residues are added at the carboxyl terminus of the $CH_1$ domain, including one or more cysteines from the antibody hinge region.

The Fab'-SH refers to a Fab' in which the cysteine residue(s) of the constant domain possesses a free thiol group.

The Fv is the smallest antibody fragment containing a complete antigen-recognition and antigen-binding sites, consisting of a dimer in which one heavy chain variable region and one light chain variable region are tightly and non-covalently associated. These two regions fold to form six hypervariable loops (three loops each from the heavy and light chains), and the loops provide the amino acid residues for antigen binding and confer antigen-binding specificity to the antibody. However, even a single variable region has the ability to recognize and bind to an antigen, although with lower affinity than the entire binding site.

The single-chain antibody scFv is an antibody fragment comprising VH and VL antibody domains linked by a single polypeptide chain; preferably, the scFv polypeptide may further comprise a polypeptide linker between the VH and VL domains that allows the scFv to form a desired structure for antigen binding. Herein, it may also be referred to as an scFv antibody fragment, an antigen-binding fragment scFv, an scFv antibody, an antibody scFv, or simply scFv.

The $F(ab')_2$ fragment is generated as a pair of Fab' fragments via a hinge cysteine between the Fab' fragments.

The diabody refers to a small antibody fragment prepared by constructing an scFv fragment using a short linker (about 5-10 residues) between the VH and VL domains, such that intra-chain pairing of the V domains is achieved rather than inter-chain pairing, thereby forming a bivalent fragment, i.e., a fragment with two antigen-binding sites. A bispecific diabody is a heterodimer consisting of two "cross-linked" scFv fragments in which the VH and VL domains of two antibodies are present on different polypeptide chains.

The triabody and the tetrabody comprise three and four polypeptide chains, respectively, and form three and four antigen-binding sites, respectively, which may be identical or different.

The minibody, also called $(scFv\text{-}CH_3)_2$, is a dimeric molecule consisting of two polypeptide chains, each comprising an scFv molecule (e.g., a modified scFv molecule including the altered VH domain described above) fused to a $CH_3$ domain or a portion thereof through a linking peptide.

The scFv-Fc refers to a polypeptide in which an scFv and an Fc are linked.

The $(scFv)_2$-Fc refers to a polypeptide in which two types of scFv and an Fc are linked.

The Fynomer refers to a non-immunoglobulin-derived binding polypeptide derived from the human Fyn SH3 domain. Fyn SH3-derived polypeptides are well-known in the art and are described, for example, in D Grabulovski et al. (2007), Journal of Biological Chemistry, Volume 282, Issue 5, 2007, pp. 3196-3204, and WO 2008/022759. A Fynomer can be genetically fused with other molecules (e.g., antibodies) to generate a "FynomAb", a form that can be engineered to have dual specificity.

The dual-affinity re-targeting (DART) protein and TRIDENT refer to engineered molecules designed to bind simultaneously to two or more targets. A DART refers to a covalently linked bispecific diabody, such as a diabody linked via a C-terminal disulfide bridge, with its specific structure and definition described, for example, in S. Johnson et al., Journal of Molecular Biology, Volume 399, Issue 3, 2010, pp. 436-449.

The anticalin is a type of antibody mimetic and is a family of molecules related to human lipocalins. The characteristics and types of anticalins are described in A. Skerra, Current Opinion in Biotechnology, Volume 18, Issue 4, 2007, pp. 295-304.

The $FN_3$ monobody is a synthetic binding protein constructed using the fibronectin type III ($FN_3$) domain as a molecular scaffold. The monobody was first introduced in 1998 by the Koide group as an alternative to antibodies for target-binding proteins (Koide et al., Journal of Molecular Biology, Volume 284, Issue 4, 1998, pp. 1141-1151).

The DARPin (Designed Ankyrin Repeat Protein) refers to an ankyrin repeat domain (166 residues) designed to provide a rigid interface typically arising from three repeated β-turns. DARPins generally have three repeats corresponding to an artificial consensus sequence, with six positions per repeat being randomized.

The Affibody refers to a family of antibody mimetics derived from the Z-domain of Staphylococcal protein A. Structurally, the Affibody molecule is based on a three-helix bundle domain, which can also be included in fusion proteins. By itself, an Affibody has a molecular weight of approximately 6 kDa and is stable at high temperatures and under acidic or alkaline conditions. Target specificity is obtained by randomizing 13 amino acids located in the two alpha-helices involved in the binding activity of the parent protein domain (Feldwisch, J., Tolmachev, V. (2012). Engineering of Affibody Molecules for Therapy and Diagnostics. In: Voynov, V., Caravella, J. (eds) Therapeutic Proteins. Methods in Molecular Biology, vol 899. Humana Press, Totowa, NJ.).

The Affilin refers to an antibody mimetic developed by using gamma-B crystallin or ubiquitin as a scaffold and modifying the amino acids on the surface of these proteins by random mutagenesis. The selection of Affilins with a desired target specificity is performed, for example, by phage display or ribosome display technology.

The Affimer represents an evolution of peptide aptamers. Affimers are small, highly stable proteins engineered to display peptide loops that provide a high-affinity binding surface for a specific target protein or antigen. Affimers can have the same advantages of antibody specificity but have the benefit of being smaller, capable of being chemically synthesized or modified, and free from cell culture contaminants. Affimers are low-molecular-weight proteins, typically 12 to 14 kDa, derived from the cystatin family of cysteine protease inhibitors. The Affimer scaffold is a stable protein based on the cystatin protein fold. It displays two peptide loops and an N-terminal sequence that can be randomized to bind to different target proteins with high affinity and specificity.

The Affitin, also known as Nanofitin, is an antibody mimetic protein derived from the DNA-binding protein Sac7d of *Sulfolobus acidocaldarius.* Affitins generally have a molecular weight of approximately 7 kDa and are designed to specifically bind to target molecules by randomizing amino acids on the binding surface (B Mouratou et al. Biomolecules. 2015 Jan 30;5(1), pp. 60-75).

The Alphabody refers to small 10 kDa proteins engineered to bind to various antigens. Alphabodies are developed as scaffolds with a set of amino acid residues that can be modified to bind protein targets while maintaining correct folding and thermal stability. The Alphabody scaffold is computationally designed based on a double coil structure but has no known natural counterpart. Initially, the scaffold was made of three peptides that non-covalently associated to form a parallel double coiled-coil trimer (US Patent Publication No. 20100305304), but it was later redesigned into a single polypeptide chain containing three α-helices linked by linker regions (J Desmet et al. Nature Communications volume 5, Article number: 5237 (2014)).

The Avimer refers to a class of antibody mimetics consisting of two or more peptide sequences, each with 30 to 35 amino acids, derived from the A-domains of various membrane receptors and connected by linker peptides. Binding of target molecules occurs through the A-domains, and domains with the desired binding specificity can be selected, for example, by phage display technology. The binding specificities of the different A-domains contained in an Avimer can be, but are not required to be, identical (UH Weidle et al. Cancer Genomics Proteomics. 2013 Jul-Aug; 10(4), pp.155-68).

The Im7 refers to immunity protein 7, which binds to the 60 kDa toxin ColE7 colicin, produced by *E. coli.*

The VLR is an abbreviation for variable lymphocyte receptor, a molecule that governs the adaptive immune response in agnathans (jawless fish) such as hagfish and lampreys. Unlike conventional antibodies, it is a receptor belonging to the single-chain leucine-rich repeat family and has specific binding ability to a particular antigen (Boehm et al., Annual Review of Immunology Vol. 30, pp.203-220). The repebody (Lee et al., Molecular Therapy, 2014 22(7), pp. 1254-1265), a recombinant protein produced by mutating the VLR to increase affinity for a specific protein, can also be considered to belong to VLRs.

The VNAR is a key component of shark adaptive immune system and is the smallest IgG-like protein in the animal kingdom.

The Trimab refers to a tri-specific antibody.

The CrossMab is a chimeric antibody composed of halves of two full-length antibodies, prepared by combining (i) knobs-into-holes technology, which promotes correct pairing between the two heavy chains, and (ii) an exchange technique between the heavy and light chains of one of the two Fabs to introduce asymmetry that avoids light chain pairing errors.

The nanobody, also referred to as a VHH antibody or single-domain antibody fragment (sdAb), is an antibody fragment consisting of a single monomeric variable antibody domain.

The bi-nanobody is a tetramer made by chemically conjugating two types of scFvs.

The di-sdFv is an sdFv dimer in which the heavy and light chains of the sdFv are linked by a disulfide bond.

The term "vector" as used herein is used interchangeably with recombinant vector, cloning vector, and expression vector, and refers to a DNA molecule capable of self-replication in prokaryotic and/or eukaryotic cells, which is generally used as an intermediate carrier for transferring a gene or DNA fragment into a cell. A vector typically includes, but is not limited to, an origin of replication capable of replicating in prokaryotic and/or eukaryotic cells, a selectable marker gene that can confer resistance to specific conditions/substances such as an antibiotic-degrading enzyme, and a promoter that allows for the transcription of the gene in eukaryotic or prokaryotic cells.

One type of vector is a "plasmid", which refers to a circular double-stranded DNA loop into which additional DNA fragments can be ligated. Another type of vector is a viral vector, in which additional DNA fragments can be ligated into a viral genome. Certain vectors can replicate autonomously within the host cell into which they are introduced (e.g., bacterial vectors having a bacterial origin of replication and episomal mammalian vectors).

As used herein, the term "specifically binds to" or "specific for" means determinative for the presence of a target in a heterogeneous population of molecules, including biological molecules, and refers to a measurable and reproducible interaction, such as a binding between a target and an antibody. For example, an antibody that specifically binds to a particular target (e.g., an epitope) is an antibody that binds to the target with greater affinity, greater avidity, more readily, and/or with a longer duration than it binds to other targets.

As used herein, the term "anti-BCAM antibody or an antigen-binding fragment thereof that specifically binds to human BCAM protein" may refer to an antibody or an antigen-binding fragment thereof that binds to human BCAM protein with a binding dissociation equilibrium constant ($K_D$) of 1 x $10^{-6}$ M or less.

The term "$K_D$" as used herein refers to the binding equilibrium dissociation constant for a specific antibody-antigen interaction, which is calculated using the formula $K_D$ = Kd/Ka (where Ka is an association rate constant and Kd is a dissociation rate constant), and the constant $K_D$ has units of M. The $K_D$ value for an antibody can be measured using methods widely established in the art. Preferable methods for measuring the $K_D$ value of an antibody include surface plasmon resonance (SPR), preferably using a biosensor system such as a Biacore® system, or Bio-layer Interferometry (BLI), for example, using an Octet® system. In one specific example, the $K_D$ mentioned herein may be a value obtained through Bio-Layer Interferometry using an Octet® system.

The term "$EC_{50}$" as used herein refers to a term related to in vitro or in vivo assays using an antibody, and means the concentration of the antibody that induces 50% of the maximum response, i.e., the midpoint response between the maximum reaction and the baseline.

The term "operably linked" as used herein means that two DNA fragments are joined such that the amino acid sequences encoded by both DNA fragments remain in-frame.

The term "subject" as used herein includes both humans and non-human animals. Non-human animals include all vertebrates, for example, mammals and non-mammals, such as non-human primates, sheep, dogs, cats, cattle, horses, chickens, amphibians, and reptiles, but mammals such as non-human primates, sheep, dogs, cats, cattle, and horses are preferable. A preferable subject is a human in need of prevention or treatment of cancer.

As used herein, the term "comprising", "comprises", or variations thereof has an openended meaning. As an

example, an antibody or an antigen-binding fragment thereof that comprises a listed amino acid sequence may include additional amino acid sequences, whether essential or not, that are not listed. Herein, for each chain or its variable region to comprise a specific amino acid sequence means all cases of including the entire amino acid sequence, having the entire amino acid sequence, or consisting of the amino acid sequence.

As used herein, the term "consisting of" or variations thereof means that any element not mentioned or listed in the description of the respective component for an embodiment is not permitted.

## Anti-BCAM Antibody or Antigen-Binding Fragment Thereof, or Antibody-Drug Conjugate Comprising the Same

In one aspect, the present invention provides an anti-BCAM antibody or an antigen-binding fragment thereof that binds to a BCAM protein, or an antibody-drug conjugate comprising the same.

In one embodiment of the present invention, the anti-BCAM antibody or antigen-binding fragment thereof may be an anti-BCAM antibody or an antigen-binding fragment thereof comprising: a heavy chain CDR1 comprising the amino acid sequence of SEQ ID NO: 3; a heavy chain CDR2 comprising the amino acid sequence of SEQ ID NO: 4; a heavy chain CDR3 comprising the amino acid sequence of SEQ ID NO: 5; a light chain CDR1 comprising the amino acid sequence of SEQ ID NO: 6; a light chain CDR2 comprising the amino acid sequence of SEQ ID NO: 7; and a light chain CDR3 comprising the amino acid sequence of SEQ ID NO: 8.

In another embodiment of the present invention, the anti-BCAM antibody or an antigen-binding fragment thereof may be an anti-BCAM antibody or an antigen-binding fragment thereof comprising a heavy chain variable region comprising an amino acid sequence having at least 95% sequence identity (preferably, at least 96%, 97%, 98%, or 99% sequence identity) to the amino acid sequence of SEQ ID NO: 1, and a light chain variable region comprising an amino acid sequence having at least 95% sequence identity (preferably, at least 96%, 97%, 98%, or 99% sequence identity) to the amino acid sequence of SEQ ID NO: 2.

In yet another embodiment of the present invention, the anti-BCAM antibody or an antigen-binding fragment thereof may be an anti-BCAM antibody or an antigen-binding fragment thereof comprising a heavy chain variable region consisting of the amino acid sequence of SEQ ID NO: 1 and a light chain variable region consisting of the amino acid sequence of SEQ ID NO: 2.

In one embodiment of the present invention, an antibody comprising a heavy chain variable region consisting of the amino acid sequence of SEQ ID NO: 1 and a light chain variable region comprising the amino acid sequence of SEQ ID NO: 2 may be referred to as "Ab-I".

In one embodiment of the present invention, the anti-BCAM antibody may be a monoclonal antibody, a polyclonal antibody, a chimeric antibody, a multi-specific antibody (e.g., a bispecific antibody), a heteroconjugate antibody, a humanized antibody, and any other modified form of an immunoglobulin molecule containing an antigen recognition site with the required specificity, for example, glycosylation variants of the antibody, amino acid sequence variants of the antibody, and covalently modified antibodies.

Furthermore, the antigen-binding fragment of the anti-BCAM antibody may be Fab, Fab', Fab'-SH, Fv, a single-chain antibody scFv, an $F(ab')_2$ fragment, a light chain variable region (VL), a heavy chain variable region (VH), a diabody, a triabody, a tetrabody, a minibody, IgG delta $CH_2$, scFv-Fc, $(scFv)_2$-Fc, a Fynomer, a dual-affinity re-targeting protein, an anticalin, an $FN_3$ monobody, a DARPin, an Affibody, an Affilin, an Affimer, an Affitin, an Alphabody, an Avimer, Im7, VLR, VNAR, a Trimab, a CrossMab, a TRIDENT, a nanobody, a bi-nanobody, or a di-(di)-sdFv.

In one embodiment of the present invention, the anti-BCAM antibody or an antigen-binding fragment thereof may be one that specifically binds to a BCAM protein, such as a human BCAM protein.

In another embodiment of the present invention, the anti-BCAM antibody or an antigen-binding fragment thereof can bind to a BCAM protein with an $EC_{50}$ of 150 nM or less, such as 130 nM or less, 100 nM or less, 50 nM or less, 20 nM or less, or 10 nM or less, as measured by an ELISA assay. Preferably, the BCAM protein may be a human BCAM protein.

In yet another embodiment of the present invention, the anti-BCAM antibody or an antigen-binding fragment thereof can bind to human BCAM protein with a binding dissociation equilibrium constant ($K_D$) of 1 x $10^{-6}$ M or less. Preferably, the anti-BCAM antibody or an antigen-binding fragment thereof can bind to human BCAM protein with a binding dissociation equilibrium constant ($K_D$) of 1 x $10^{-6}$ M to 1 x $10^{-11}$ M, $10^{-6}$ M to 1 x $10^{-8}$ M, 1 x $10^{-7}$ M or less, 1 x $10^{-8}$ M or less, 1 x $10^{-9}$ M or less, 1 x $10^{-10}$ M or less, or 1 x $10^{-11}$ M or less.

In another embodiment of the present invention, the antibody or an antigen-binding fragment thereof of the present invention can be in a form conjugated with a drug, i.e., it can form an antibody-drug conjugate (ADC). The term "antibody-drug conjugate" or "ADC" as used herein may be represented by the formula $M-[L-D]_n$, where M represents the antibody molecule, i.e., the anti-BCAM antibody or an antigen-binding fragment thereof of the present invention, L is an optional linker or linker unit, which is a cleavable or non-cleavable linker, D is a suitable drug or prodrug, and n is from 1 to 20. Said n may be 10 or less, 9 or less, 8 or less, 7 or less, or 6 or less.

The drug included in the ADC may be appropriately selected according to therapeutic or diagnostic use, etc., as

long as it does not interfere with the specific binding of the antibody of the present invention. In one embodiment, said drug may include, but is not limited to, a cytotoxic agent (e.g., a chemotherapeutic agent), a prodrug-converting enzyme, a radioisotope or compound, or a toxin. The drugs and linkers that can be included in an ADC and their production methods can be in accordance with methods known in the art.

Furthermore, in one embodiment of the present invention, the present invention provides said antibody-drug conjugate comprising an anti-BCAM antibody or an antigen-binding fragment thereof.

## Nucleic Acid Encoding Anti-BCAM Antibody or Antigen-Binding Fragment Thereof, and Vector

One aspect of the present invention relates to a nucleic acid encoding the anti-BCAM antibody or an antigen-binding fragment thereof of the present invention.

The nucleic acid may be present in whole cells or cell lysates, or it may exist in a specifically purified or substantially pure form. The nucleic acid is an "isolated" or "substantially purely isolated" nucleic acid, purified from other cellular components or other contaminants, for example, other cellular nucleic acids or proteins, by standard techniques such as alkaline/SDS treatment, CsCl banding, column chromatography, agarose gel electrophoresis, and other methods widely known in the art.

In one embodiment of the present invention, the nucleic acid may be, for example, DNA or RNA, and may or may not contain intron sequences. In a preferable embodiment, the nucleic acid is a cDNA molecule.

In another embodiment of the present invention, the nucleic acid encodes the light chain region, the heavy chain region, or both the light and heavy chain regions of the anti-BCAM antibody or an antigen-binding fragment thereof, and preferably, may encode the light chain variable region, the heavy chain variable region, or both the light and heavy chain variable regions. Once a DNA fragment encoding the VL and/or VH region is obtained, such a DNA fragment may be further manipulated, for example, by standard recombinant DNA techniques to convert the variable region gene into a full-length antibody chain gene, a Fab fragment gene, or an scFv gene. In such a manipulation process, the VL- or VH-encoding DNA fragment is operably linked to another DNA fragment encoding another protein, for example, an antibody constant region or a flexible linker.

In one embodiment of the present invention, an isolated DNA encoding the VH region may be converted into a full-length heavy chain gene by operably linking the VH-encoding DNA to another DNA molecule encoding the heavy chain constant regions ($CH_1$, $CH_2$, and $CH_3$). The heavy chain constant region may be an IgG1, IgG2, IgG3, IgG4, IgA, IgE, IgM, or IgD constant region.

In another embodiment of the present invention, for a Fab fragment heavy chain gene, the VH-encoding DNA may be operably linked to another DNA molecule that encodes only the heavy chain $CH_1$ constant region.

In yet another embodiment of the present invention, to produce an scFv gene, the VL- and VH-encoding DNA fragments may be operably linked to another fragment encoding a flexible linker, for example, the amino acid sequence $(Gly_4\text{-}Ser)_3$, and as a result, the VL and VH sequences can be expressed as a continuous single-chain protein having VL and VH regions linked by a flexible linker.

In another aspect of the present invention, the present invention provides a recombinant expression vector encoding the anti-BCAM antibody or an antigen-binding fragment thereof, or a recombinant expression vector comprising the nucleic acid.

## Production of Anti-BCAM Antibody or Antigen-Binding Fragment Thereof

In one aspect of the present invention, the anti-BCAM antibody or an antigen-binding fragment thereof of the present invention may be produced by any suitable method known in the art to which the present invention pertains.

In one embodiment of the present invention, the anti-BCAM antibody may be produced using techniques well-established in the art, for example, hybridoma, recombinant, phage display, transfection, synthetic techniques, or a combination thereof, or other techniques readily known in the art (see, for example, Jayasena, S.D., Clin. Chem., 45: 1628-50 (1999) and Fellouse, F.A., et al, J. Mol. Biol., 373(4):924-40 (2007)).

In another embodiment of the present invention, the anti-BCAM antibody may be produced using phage display technology. Specifically, the anti-BCAM antibody may be produced using a method wherein genes encoding the heavy and light chain variable regions of human antibody may be cloned into a phagemid vector, where they are fused to a phage surface protein (pIII) and expressed in *Escherichia coli.;* infection with an M13 helper phage then allows the generation of an antibody library, in which scFv or Fab antibody fragments with various heavy and light chain variable region sequences are displayed on the surface of the phage; panning techniques with the library may then be used to isolate antibody fragments that specifically bind to a target antigen; and after characterizing the isolated antibody fragments, they may be converted into whole IgG form and expressed in mammalian cells for large-scale production of specific human monoclonal antibodies. A naive antibody library, which utilizes antibody genes already present in the human body, may be used as the phage display library. Alternatively, a synthetic antibody library, in which randomly synthesized sequences are inserted into

antibody CDR regions to enhance diversity, may be used. A phagemid vector is a plasmid DNA containing a phage origin of replication, typically including an antibiotic resistance gene as a selection marker. Phagemid vectors used in phage display generally include gene **III** (gIII) or a portion thereof from the M13 phage, and scFv genes are ligated to the 5' terminus of gIII to allow expression in transformed host cells. A helper phage provides the necessary genetic information for phagemid assembly into phage particles. As the phagemid vector contains the phage gene III or only a part thereof, the host cells transformed with the phagemid should be infected with a helper phage to supply the remaining phage genes. Various phagemids, such as M13K07 or VCSM13, exist and typically include kanamycin resistance genes to allow selection of helper phage-infected transformants. Furthermore, as phagemids have defective packaging signals, the phagemid genome is preferentially assembled into phage particles over the helper phage genome.

In another embodiment of the present invention, the anti-BCAM antibody may be produced using hybridoma technology. Specifically, after injecting a test subject (e.g., a mouse) with the BCAM antigen, hybridomas expressing antibodies with the desired sequence or functional properties may be isolated, thereby producing the antibody, for example, in the form of a monoclonal antibody.

In one embodiment of the present invention, the anti-BCAM antibody may be produced in the form of a monoclonal antibody, and the nucleotide sequence encoding the monoclonal antibody may be immediately isolated and sequenced using conventional methods (e.g., using oligonucleotide probes that can specifically bind to the genes encoding the heavy and light chains of the monoclonal antibody), and hybridoma cells can be used as a preferable source of the nucleotide sequence. At this time, once the nucleotide sequence is isolated, it can be placed into an expression vector and then transfected into host cells, for example, *E. coli* cells, simian COS cells, Chinese hamster ovary (CHO) cells, or myeloma cells that do not otherwise produce immunoglobulin protein, to obtain the synthesis of the monoclonal antibody in recombinant host cells.

In another aspect of the present invention, the present invention provides an isolated host cell for recombinantly producing the anti-BCAM antibody or an antigen-binding fragment thereof.

In one embodiment of the present invention, the anti-BCAM antibody or an antigen-binding fragment thereof may be expressed by introducing the nucleic acid into any recombinant expression system, including bacterial, yeast, insect, or mammalian systems. The recombinant expression system may include the isolated host cell.

In another embodiment of the present invention, the expression system may be an isolated mammalian host cell such as, but not limited to, CHO cells, DG44 or DUXB11 cells, NS0 myeloma cells, monkey kidney cell lines (e.g., CV1 cells, COS cells, etc.), SP2 cells, human embryonic kidney (HEK) cells, Chinese hamster fibroblasts, human cervical carcinoma cells (e.g., HELA), murine fibroblasts (e.g., BALBc/3T3), murine myeloma cells (P3x63-Ag3.653; NS0; SP2/O), hamster kidney cells (e.g., HAK), murine L cells (e.g., L-929), human lymphocytes (e.g., RAJI), human kidney cells (e.g., 293 and 293T).

In another aspect of the present invention, the present invention provides a method for producing the antibody or an antigen-binding fragment thereof, the method comprising: culturing the host cell under conditions that allow for the production of the antibody or antigen-binding fragment thereof, and isolating the antibody or antigen-binding fragment thereof from the host cell or culture medium.

In one embodiment of the present invention, after a recombinant expression vector is introduced into a recombinant expression system, host cells are cultured under conditions sufficient for the antibody to be expressed within the cells or secreted into the culture medium, and then the antibody or an antigen-binding fragment thereof is then recovered from the host cells or the culture medium using standard protein purification methods.

In another embodiment of the present invention, the recombinant expression vector can be transfected into host cells by standard techniques, at which time known techniques used for the intracellular introduction of exogenous nucleic acids, such as electroporation, calcium-phosphate precipitation, and DEAE-dextran transfection, can be used.

## Uses of Anti-BCAM Antibody or Antigen-Binding Fragment Thereof and Anti-BCAM Antibody-Drug Conjugate

The antibody and antibody-drug conjugate of the present invention can be usefully applied to various purposes, including but not limited to, therapeutic treatment methods and diagnostic methods.

In one aspect of the present invention, the antibody or an antigen-binding fragment thereof, or the antibody-drug conjugate may specifically bind to a BCAM protein and be used for the prevention, amelioration, or treatment of a disease associated with BCAM function or expression.

In another embodiment of the present invention, the disease associated with BCAM function or expression may be cancer. By specifically binding to the BCAM protein, the anti-BCAM antibody or an antigen-binding fragment thereof, or the antibody-drug conjugate of the present invention can effectively bind to BCAM-expressing cancer cells, thereby inhibiting the growth of cancer cells *in vivo,* and can be usefully employed for the prevention, amelioration, or treatment of cancer. BCAM is known to be involved in tumor migration, and furthermore, its overexpression is known to appear particularly in skin cancer, ovarian cancer, pancreatic cancer, breast cancer, etc. (see, e.g., F.R.M. Latini et al., Blood Cells, Molecules and Diseases 50 (2013), pp. 161-165). Accordingly, the anti-BCAM antibody of the present invention may be

used for any cancer in which tumor migration is observed, such as metastatic cancer, and may also be used for other specific cancer types in which overexpression of BCAM is observed.

Examples of cancers whose growth can be inhibited when using the anti-BCAM antibody, an antigen-binding fragment thereof or the antibody-drug conjugate of the present invention include, but are not limited to, gallbladder cancer, melanoma (e.g., metastatic malignant melanoma, malignant melanoma of the skin and intraocular), kidney cancer (e.g., clear cell carcinoma, renal cell carcinoma), prostate cancer (e.g., hormone-refractory prostate adenocarcinoma), breast cancer (e.g., invasive breast cancer, non-invasive breast cancer), colon cancer, rectal cancer, colorectal cancer, colon cancer, lung cancer (e.g., non-small cell lung cancer (NSCLC), small cell lung cancer), bone cancer, pancreatic cancer, liver cancer (e.g., hepatocellular carcinoma), skin cancer, head or neck cancer, uterine cancer, ovarian cancer, anal cancer, stomach cancer, testicular cancer, fallopian tube carcinoma, endometrial carcinoma, cervical carcinoma, vaginal carcinoma, vulvar carcinoma, Hodgkin's disease, non-Hodgkin's lymphoma, throat cancer, laryngeal cancer, esophageal cancer, oral cancer, tongue cancer, small intestine cancer, endocrine system cancer, thyroid cancer, parathyroid cancer, adrenal cancer, soft tissue sarcoma, urethral cancer, penile cancer, bone marrow cancer, chronic and acute leukemias (e.g., acute myeloid leukemia (AML), chronic myeloid leukemia, acute lymphoblastic leukemia, chronic lymphocytic leukemia), pediatric solid tumors, lymphocytic lymphoma, bladder cancer, ureteral cancer, renal pelvis carcinoma, neoplasia of the central nervous system (CNS), primary CNS lymphoma, tumor neovascularization, spinal axis tumors, brainstem glioma, pituitary adenoma, Kaposi's sarcoma, epidermoid carcinoma, squamous cell carcinoma, T-cell lymphoma, environmentally induced cancers (e.g., asbestos-induced cancer), brain cancer, glioma, hypopharyngeal squamous cell carcinoma, glioblastoma, neuroblastoma, and urothelial carcinoma.

In one embodiment of the present invention, the present invention provides a method for the prevention, amelioration, or treatment of cancer, comprising administering an effective amount of an anti-BCAM antibody or an antigen-binding fragment thereof, or an anti-BCAM antibody-drug conjugate to a subject.

In another embodiment of the present invention, the present invention provides a use of an anti-BCAM antibody or an antigen-binding fragment thereof, or an anti-BCAM antibody-drug conjugate for the prevention, amelioration, or treatment of cancer.

In another aspect of the present invention, the present invention provides a pharmaceutical composition for the prevention, amelioration, or treatment of cancer, comprising an anti-BCAM antibody or an antigen-binding fragment thereof, or an anti-BCAM antibody-drug conjugate. The anti-BCAM antibody or an antigen-binding fragment thereof or the anti-BCAM antibody-drug conjugate may be included in the pharmaceutical composition in an effective amount.

The anti-BCAM antibody or an antigen-binding fragment thereof, or the antibody-drug conjugate of the present invention may be used alone or in combination with other anticancer therapies. Other anticancer therapies may include, for example, standard cancer therapies (e.g., chemotherapy, radiation therapy, or surgery); or other anticancer agents, such as cytotoxic agents, cytostatic agents, antihormonal agents, anti-angiogenesis or antimetabolite agents, targeted anticancer agents, immune stimulators or immunomodulators, immune checkpoint inhibitors, or antibodies conjugated to cytotoxic agents, cytostatic agents, and other toxic agents.

Preferably, the anti-BCAM antibody or an antigen-binding fragment thereof, or the anti-BCAM antibody-drug conjugate of the present invention may be used in combination with other anticancer agents, such as immune checkpoint modulators, chemotherapeutic agents, or radiation therapy. The immune checkpoint modulator may be, for example, an anti-CTLA-4 antibody (e.g., ipilimumab), an anti-PD-1 antibody (e.g., pembrolizumab, nivolumab), or an anti-PD-L1 antibody (e.g., atezolizumab, avelumab, durvalumab). The chemotherapeutic agents include, but are not limited to, alkylating agents, antimetabolites, kinase inhibitors, spindle toxin plant alkaloids, cytotoxic/antitumor antibiotics, topoisomerase inhibitors, photosensitizers, anti-estrogens and selective estrogen receptor modulators (SERMs), anti-progestins, estrogen receptor down-regulators (ERDs), estrogen receptor antagonists, luteinizing hormone-releasing hormone agonists, anti-androgens, aromatase inhibitors, EGFR inhibitors, VEGF inhibitors, and antisense oligonucleotides that inhibit the expression of genes involved in abnormal cell proliferation or tumor growth. Specific examples of the chemotherapeutic agents of the present invention include Gemcitabine, Vinorelbine, Etoposide (VP-16), platinum analogues such as cisplatin or carboplatin, and taxoids such as paclitaxel, albumin-bound paclitaxel, or docetaxel.

When the anti-BCAM antibody or an antigen-binding fragment thereof, or the antibody-drug conjugate of the present invention is used in combination with other anticancer agents, they may be administered separately or as a combination product in which multiple active ingredients are present in a single pharmaceutical formulation. If administered as separate formulations, the two formulations may be administered sequentially or simultaneously. In the case of simultaneous administration, they are provided to the subject together. In the case of sequential administration, they may be provided with a time interval that is not long (e.g., may be administered to the subject within a period of 12 hours or less, or 6 hours or less), and may be administered to the subject at intervals of 1 day, 2 days, 3 days, 4 days, 5 days, 6 days, 1 week, 2 weeks, 3 weeks, or 4 weeks or more.

The pharmaceutical composition may include inactive ingredients, i.e., pharmaceutically acceptable excipients (see, e.g., Handbook of Pharmaceutical Excipients). The pharmaceutical composition may be prepared, for example, by mixing with a physiologically acceptable carrier, excipient, or stabilizer in the form of a lyophilized powder, slurry, aqueous

solution, or suspension.

Suitable routes of administration for the pharmaceutical composition include parenteral administration, for example, intramuscular, intravenous, or subcutaneous administration. Administration of the antibody to use for the pharmaceutical composition of the present invention or to practice the method of the present invention may be carried out by various conventional methods such as topical application or skin, subcutaneous, intraperitoneal, parenteral, intra-arterial, or intravenous injection. In one embodiment, the antibody of the present invention is administered intravenously or subcutaneously.

The pharmaceutical composition may further comprise an anticancer agent. In one aspect, the pharmaceutical composition may be used in combination with an additional anticancer agent. The anticancer agent may be administered simultaneously with the antibody or an antigen-binding fragment thereof or the antibody-drug conjugate in a single formulation, or simultaneously or sequentially in separate formulations, and the type and administration method of the anticancer agent are as described above.

In one embodiment, the present invention provides a method for the prevention, amelioration, or treatment of cancer, comprising administering to a subject an effective amount of an anti-BCAM antibody or an antigen-binding fragment thereof or an antibody-drug conjugate in combination with an additional anticancer agent. This embodiment includes administering the anti-BCAM antibody or antigen-binding fragment together with an additional anticancer agent in a single composition for simultaneous administration, as well as administering compositions in which each is separately included to a subject in need thereof, either simultaneously or sequentially. At this time, the routes of administration include parenteral administration, for example, intramuscular, intravenous, or subcutaneous administration.

In another aspect of the present invention, the present invention provides a use of an anti-BCAM antibody or an antigen-binding fragment thereof, or an anti-BCAM antibody-drug conjugate for combination therapy with an additional anticancer agent for the prevention, amelioration, or treatment of cancer.

In one aspect of the present invention, the present invention provides a pharmaceutical composition or combination for the prevention, amelioration, or treatment of cancer, comprising an anti-BCAM antibody or an antigen-binding fragment thereof or an anti-BCAM antibody-drug conjugate, and an additional anticancer agent. Herein, a pharmaceutical composition or combination comprising an anti-BCAM antibody or an antigen-binding fragment thereof, and an additional anticancer agent includes not only cases where the two components are physically present together in a single formulation, but also cases where they are administered simultaneously or sequentially as separate formulations, at which time the two drugs may be provided individually or together in a single kit. Accordingly, the present invention provides a kit for the prevention, amelioration, or treatment of cancer, comprising an anti-BCAM antibody or an antigen-binding fragment thereof or an anti-BCAM antibody-drug conjugate, and an additional anticancer agent.

In another aspect of the present invention, the present invention provides a use of the anti-BCAM antibody or an antigen-binding fragment thereof or the anti-BCAM antibody-drug conjugate for the manufacture of a medicament for the treatment of a disease associated with the function or expression of BCAM.

## Examples

Hereinafter, the present invention will be described in more detail through examples. These examples are intended only to explain the present invention more specifically, and it will be apparent to those of ordinary skill in the art that the scope of the present invention is not limited by these examples according to the gist of the present invention.

### **Example** 1: **Production of BCAM-Specific Antibody via Phage Display**

### 1.1. Construction and Selection of scFv Antibody Library via Phage Display

The antibody of the present invention was produced through the phage display method.

The mRNA of the heavy and light chain variable regions of antibodies obtained from human blood or bone marrow was amplified by PCR to synthesize cDNA. The cDNA was cloned into a phagemid vector using a restriction enzyme and then expressed in *E. coli* by electroporation. Subsequently, a helper phage was infected to prepare a human library in scFv format.

The library was screened for antibodies that bind with high affinity to the target antigen, BCAM, through biopanning. Positive clones that bind to human BCAM protein were selected, and BCAM-specific scFv sequences were selected through sequencing.

### 1.2. Production of Ab-I Antibody

To produce an antibody in the human IgG4 format with an S228P mutation, the amino acid sequences of the light and heavy chain regions of the scFv selected in Example 1.1 were synthesized after being cloned into DNA sequences,

and each was cloned into a pcDNA3.4 expression vector. Using this vector, transient transfection was performed in the ExpiCHO-S cell line, followed by incubation for 8 days to allow for antibody expression. The obtained culture supernatant was subjected to primary purification using affinity chromatography with Protein A, eluted with a low-pH buffer, and then formulated to produce the anti-BCAM human IgG4 antibody, Ab-I, with a purity of 97% (by SEC-HPLC).

The heavy and light chain variable regions of the produced Ab-I antibody are shown in FIG. 1 and Table 1, and the amino acid sequences of the heavy and light chain CDRs of the Ab-I antibody are shown in Table 2. The CDRs were defined based on the IMGT CDR and Martin CDR standards (in Table 1 and FIG. 1, the regions marked in bold or underlined indicate each CDR).

**[TABLE 1]**

| SEQ ID NO | Region | Sequence |
|---|---|---|
| SEQ ID No.1 | Heavy chain variable regions (VH) | EVQLVESGGGLVKPGGSLRLSCAAS**GFTFSSYSMN**WVRQAPGKGLEWVS**SISSSSSYIYYADSVKG**RFTISRDNAKNSLYLQMNSLRAEDTAVYYC**ARIIGGWLGDY**WGQGTLVTVSS |
| **SEQ ID NO** | **Region** | **Sequence** |
| SEQ ID No.2 | Light chain variable regions (VL) | NIQMTQSPSSVSASVGDRVTITC**RASQGISSWLA**WYQQKPGKAPKLLIY**AASSLRS**GVPSRFSGSGSGTDFTLTISSLQPEDFATYYC**QQANSFPPPT**FGGGTKVEIK |

**[TABLE 2]**

| SEQ ID NO | Region | Sequence |
|---|---|---|
| SEQ ID No.3 | VH CDR1 | GFTFSSYSMN |
| SEQ ID No.4 | VH CDR2 | SISSSSSYIYYADSVKG |
| SEQ ID No.5 | VH CDR3 | ARIIGGWLGDY |
| SEQ ID No.6 | VL CDR1 | RASQGISSWLA |
| SEQ ID No.7 | VL CDR2 | AASSLRS |
| SEQ ID No.8 | VL CDR3 | QQANSFPPPT |

**Example 2: Confirmation of Binding Affinity of Ab-I Antibody to BCAM**

**2.1. Confirmation of Binding Ability to BCAM by ELISA**

An ELISA (Enzyme-Linked Immunosorbent Assay) test was conducted to confirm the ability of the Ab-I antibody produced in Example 1 to bind to human BCAM protein. An antigen protein (human BCAM protein, 10238-H08H, SinoBiological, China) was diluted in PBS buffer to a concentration of 25 nM, and 50 μL was coated into each well of a 96-well half plate (Costar, USA, Cat. No. 3690), followed by overnight incubation at 4°C. The next day, all solutions were removed from the plate, blocking buffer (3% BSA in PBS) was added to each well, and the plate was incubated at 37°C for 1 hour. The Ab-I antibody sample was serially diluted in blocking buffer from 1 μM in a 3-fold dilution series across 12 points down to 6 pM. After the blocking process was complete, the buffer was removed from the wells, and 50 μL of the prepared diluted antibody was added to each well and incubated at 37°C for 2 hours. After washing with 0.1% PBST (0.1% Tween 20 in PBS), a secondary antibody for detection, HRP-conjugated anti-human IgG Fc antibody (ThermoFisher Scientific, USA, Cat. No.: 31423), was added and treated at 37°C for 1 hour, followed by another wash with 0.1% PBST. For color development, 50 μL of TMB solution (ThermoFisher Scientific, USA, Cat. No.: 34028) was added to each well and allowed to react for 10 minutes at room temperature. The optical density at 450 nm ($OD_{450}$) was then measured, and the $OD_{450}$ values according to the antibody concentration are shown in FIG. 2 and Table 3. To represent the degree of binding affinity of the Ab-I antibody for the antigen protein, the $EC_{50}$ concentration was calculated, and the results are shown in Table 4.

Consequently, through the ELISA experiment, it was confirmed that the tested Ab-I antibody has excellent binding ability to human BCAM protein.

**[TABLE 3]**

| Concentration of Ab-I (nM) | $OD_{450}$ |
|---|---|
| 1000 | 2.457 ± 0.04 |
| 333 | 2.479 ± 0.01 |
| 111 | 2.527 ± 0.07 |
| 37 | 2.584 ± 0.03 |
| 12.3 | 2.698 ± 0.02 |
| 4.11 | 2.754 ± 0.00 |
| 1.37 | 2.663 ± 0.04 |
| 0.457 | 2.294 ± 0.02 |
| 0.152 | 1.514 ± 0.00 |
| 0.051 | 0.756 ± 0.01 |
| 0.017 | 0.330 ± 0.00 |
| 0.006 | 0.160 + 0.00 |

**[TABLE 4]**

| **Antibody** | Ab-I |
|---|---|
| **$EC_{50}$(nM)** | 0.124 |

**2.2. Confirmation of Binding Ability to BCAM by FACS**

A FACS (Fluorescence Activated Cell Sorting, Flow Cytometry) test was conducted to confirm the binding ability of the Ab-I antibody produced in Example 1 to the antigen protein (human BCAM protein) expressed on the cell surface.

Human BCAM antigen protein was expressed in HEK293FT cells through transfection using a 293FT/BCAM plasmid. $1x10^5$ transfected HEK293FT cells were suspended in PBS, and 50 μL thereof was seeded into a plate.

Separately, the Ab-I antibody of Example 1 and an isotype control (human IgG4) antibody were serially diluted 12 times via 3-fold dilutions in PBS, starting from a concentration of 111 nM, and were added to the plate and incubated for an appropriate time and at an appropriate temperature. PBS buffer was used as a negative control.

Afterward, the plate was washed, and Goat Anti-Human IgG (H+L) Cross-Adsorbed Secondary Antibody, Alexa Fluor™ 647 (ThermoFisher Scientific, USA, Cat. No.: A21445) (1:400) was added, and the plate was incubated again. It was then washed again with PBS, and the degree of antigen-antibody binding was analyzed using a flow cytometer.

The results are shown in Table 5 and FIG. 3.

Consequently, through the FACS experiment, it was confirmed that the Ab-I antibody has excellent binding ability to human BCAM expressed on the cell surface.

**[TABLE 5]**

| **Antibody** | **Ab-I** |
|---|---|
| $EC_{50}$(nM) | 0.594 |
| $EC_{50}$(μg/mL) | 0.086 |

**2.3. Confirmation of Binding Ability to Antigen Protein by Bio-layer Interferometry (BLI)**

A binding affinity confirmation test according to BLI for the Ab-I antibody prepared in Example 1 was conducted using an Octet (Octet® R8, Sartorius).

Specifically, to immobilize the human BCAM protein (antigen) using Amine coupling, Reactive 2nd Generation (AR2G) biosensors (Cat. No. 18-5094, Sartorius) were mounted and activated using an AR2G Reagent Kit (Cat. No. 18-5095). Subsequently, the biosensor was immersed in the antigen solution for 10 minutes to immobilize the human BCAM protein, and the reaction was terminated by immersing it in an ethanolamine solution for 5 minutes. The biosensor with the immobilized human BCAM protein was placed in a buffer solution containing the prepared antibody, and the

association reaction was monitored for 5 minutes, followed by observation of the dissociation reaction for 5 minutes. At this time, the antibody solution was serially diluted to confirm the association and dissociation rates at different concentrations. After setting the dissociation phase to 100 seconds for optimal fitting, the association (Ka) and dissociation (Kd) rate constants were determined by fitting the data to a 1:1 binding model using curve-fitting software. The binding dissociation equilibrium constant ($K_D$) was calculated as $K_D$ = Kd/Ka. The binding kinetics data are shown in Table 6 below, and the binding sensor-gram is shown in FIG. 4.

**[TABLE 6]**

| Analyte | Ligand | Ka (1/Ms) | Kd (1/s) | $K_D$(M) |
|---|---|---|---|---|
| **Ab-I** | Human BCAM | 2.18 X $10^6$ | 2.16 X $10^{-3}$ | 9.91 X $10^{-10}$ |

From this, it was confirmed that the antibody of the present invention binds to human BCAM protein with high affinity.

### Example 3: Confirmation of Antibody's Cellular Internalization into Cancer Cells through Confocal Laser Scanning Microscopy Observation

An experiment was conducted to confirm the cellular internalization of the antibody using the Ab-I antibody prepared in Example 1.

2.5 x $10^4$ SK-BR-3 cells (human breast cancer cell line, KCLB) were plated on a cell culture chamber slide (SPL, Korea). The next day, after confirming cell attachment to the plate, the existing cell culture medium was removed. The Ab-I antibody prepared in Example 1 was diluted in PBS to prepare a 10 μg/mL solution, and 500 μl was added to each well. After reacting the antibody at 4°C for 1 hour, the residual antibody was removed, and the cell slides were washed three times with PBS. The cell slides were divided into two groups: one group was used as a negative control for cellular internalization, and the other group was used as an internalization induction group. The cell slides to be used as the negative control were fixed using 4% PFA (paraformaldehyde; Sigma, USA) (Fixation, treated at room temperature for 10 minutes), and the cell slides to be used for the internalization induction group were moved to a 37°C incubator and incubated for 1 hour, followed by fixation using 4% PFA in the same manner as the negative control. After fixation was complete, the PFA was completely removed from each cell slide by washing with PBS. Then, 0.1% Triton X-100 was applied for 15 minutes at room temperature to prepare for cell membrane permeabilization for the secondary antibody. After washing with PBS and a blocking process (treating with 3% BSA for 20 minutes at room temperature), a fluorescence-conjugated secondary antibody, Goat anti-human IgG (H+L) Cross-Adsorbed Secondary Antibody, Alexa Fluor™ 488 (ThermoFisher Scientific, USA, Cat.No.: A11013) at 10 μg/mL, was applied for 1 hour at 4°C. The cell slides, with the secondary antibody treatment completed, were treated with a DAPI staining solution (Abcam, Cat.No.: ab228549) for 3 minutes at room temperature for nuclear staining. After washing with PBS, a mounting solution (Agilent Technologies, USA, Cat. No.: S3023) was applied, and the slides were covered with a cover glass to complete the slide specimens. The degree of cellular internalization of the antibody was observed under a confocal laser scanning microscope (LSM 700, Carl Zeiss, Germany) at 400x magnification.

The results are shown in FIG. 5. In the case of the negative control group (4°C, 1-hour antibody treatment condition without inducing cellular internalization, top photo in FIG. 5), the Ab-I antibody was bound to the surface of the cancer cells. However, in the case of the cellular internalization induction condition of 37°C, 1-hour antibody treatment (bottom photo in FIG. 5), it was confirmed that each antibody was internalized into the cell, resulting in a decrease in the fluorescence signal on the cell surface, whereas the fluorescence signal inside the cell increased. These results signify that the antibody of the present invention has the potential to deliver a cytotoxic drug into cells via cellular internalization, and show that it can be used in the form of an antibody-drug conjugate (ADC).

### Example 4: Preparation of Antibody-Drug Conjugate (Ab-II) using Ab-I Antibody

#### 4.1. Production of ((2R,3R)-3-((S)-1-((3R,4S,5S)-4-((S)-2-((S)-2-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)(methyl)amino)-3-methylbutanamido)-N,3-dimethylbutanamido)-3-methoxy-5-methylheptanoyl)pyrrolidin-2-yl)-3-methoxy-2-methylpropanoyl)-L-phenylalanine (Compound 1)

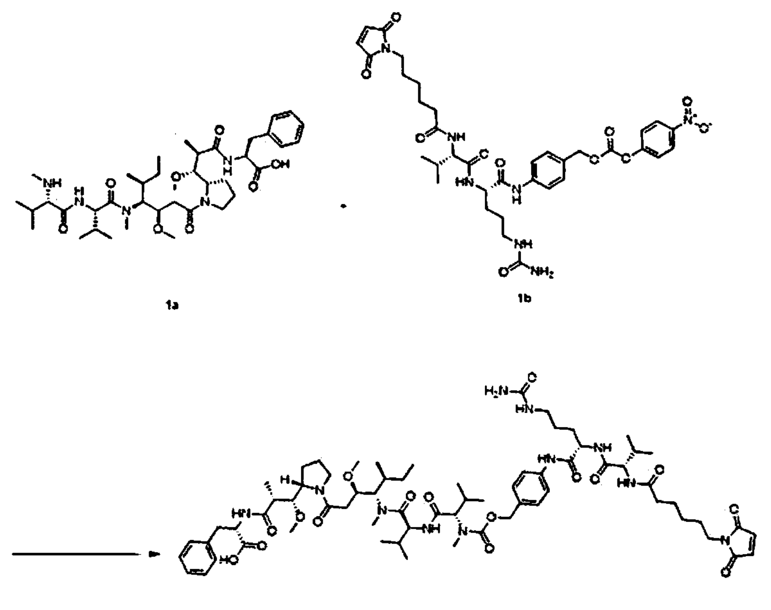

1a 1b

1

151 mg (0.205 mmol) of 4-((*S*)-2-((*S*)-2-(6-(2,5-dioxo-2,5-dihydro-1*H*-pyrrol-1-yl)hexanamido)-3-methylbutanamido)-5-ureidopentanamido)benzyl (4-nitrophenyl) carbonate (Compound 1b) was dissolved in 10 mL of *N,N*-dimethylformamide, and 22 mg (0.16 mmol) of 1-hydroxybenzotriazole was added, followed by stirring at room temperature for 30 minutes. To the reaction mixture, 100 mg (0.137 mmol) of ((2*R*,3*R*)-3-((*S*)-1-((3*R*,4*S*,5*S*)-4-((*S*)-*N*,3-dimethyl-2-((S)-3-methyl-2-(methylamino)butanamido)butanamido)-3-methoxy-5-methylheptanoyl)pyrrolidin-2-yl)-3-methoxy-2-methylpropanoyl)-L-phenylalanine **(Compound 1a),** 3.78 mL (46.8 mmol) of pyridine, and 148 mg (1.15 mmol) of diisopropylethylamine were added, followed by stirring at room temperature for 20 hours. After the reaction was complete, the reaction mixture was distilled under reduced pressure, and the obtained residue was purified using a high-performance liquid chromatography system. The obtained residue was freeze-dried to yield 40 mg of the title compound (yield: 22%).

HRMS ($ESI^+$): m/z = 1330.7665 $[M+H]^+$.

### 4.2. Production of Ab-II Antibody-Drug Conjugate

The antibody-drug conjugate Ab-II was produced by conjugating Compound 1, prepared in Example 4.1, to the antibody Ab-I from Example 1. In the present invention, a conjugation technique using the maleimide functional group present on the linker and the antibody cysteine residue side chains was used, and a method of partially reducing the disulfide bonds of the antibody was applied to activate the necessary side chains. Specifically, the antibody was partially reduced by adding the reducing agent DTT to a concentration of 2 mM under pH 8.0 conditions and reacting at 37°C for 1 hour. Subsequently, the reducing agent was removed using Amicon ultra (Millipore), and the buffer was exchanged to 100 mM Phosphate (pH 6.5). Then, Compound 1 dissolved in DMSO was added at 15 equivalents to proceed with the conjugation reaction. At this time, the concentration of the antibody during the reaction was diluted and adjusted to be 5 mg/mL, and the reaction was carried out for 1 hour using a 25°C chamber. The reaction material was purified using size-exclusion chromatography (SEC). The finally produced Ab-II was formulated in PBS.

Purity analysis of Ab-II was conducted via SEC-HPLC. Specifically, 20 μg of Ab-II was loaded onto an SEC-HPLC column, and the analysis was conducted under conditions where a 50 mM sodium phosphate buffer (pH 6.8) containing 150 mM sodium chloride was applied at a flow rate of 1 mL/min, and detection was performed using a UV detector at 214 nm. The analysis results confirmed that the purity of the produced Ab-II was 92%.

UV spectrophotometry was applied to measure the concentration of the product using the absorbance at 280 nm, and the drug-to-antibody ratio (DAR) was also calculated using the absorbance ratio at 248 nm. The final analysis resulted in a calculated DAR of 5.3 for Ab-II.

### Example 5: Confirmation of Binding Ability of Ab-II Antibody-Drug Conjugate to BCAM by ELISA

An ELISA (Enzyme-Linked Immunosorbent Assay) test was conducted to confirm the ability of the Ab-II antibody-drug conjugate produced in Example 4 to bind to human BCAM protein. An antigen protein (human BCAM protein, 10238-H08H, SinoBiological, China) was diluted in PBS buffer to a concentration of 7 nM, and 50 μL was coated into each well of a 96-well plate (9018, Corning, USA), followed by incubation at room temperature for 2 hours. All solutions were removed from the plate, blocking buffer (3% BSA in PBS) was added to each well, and the plate was incubated at 37°C for 1 hour. The Ab-II antibody-drug conjugate sample was serially diluted in blocking buffer from 100 nM in a 3-fold dilution series across 11 points down to 1.7 pM. After the blocking process was complete, the buffer was removed from the wells, and 50 μL of the prepared diluted antibody was added to each well and incubated at 37°C for 1 hour. After washing with 0.1% PBST (0.1% Tween 20 in PBS), a secondary antibody for detection of the antibody-drug conjugate (HRP-conjugated anti-human IgG Fc antibody (109-035-098, Jackson ImmunoResearch Inc., USA)), was added and treated at 37°C for 1 hour, followed by another wash with 0.1% PBST. For color development, 50 μL of TMB solution (ThermoFisher Scientific, USA, Cat. No.: 34028) was added to each well and allowed to react for 10 minutes at room temperature. The optical density at 450 nm ($OD_{450}$) was then measured, and the $OD_{450}$ values according to the antibody concentration are shown in FIG. 6. To represent the degree of binding affinity of the Ab-II antibody-drug conjugate for the antigen protein, the $EC_{50}$ concentration was calculated, and the results are shown in Table 7.

Consequently, through the ELISA experiment, it was confirmed that the tested Ab-II antibody-drug conjugate has excellent binding ability to human BCAM protein.

**[TABLE 7]**

| Antibody-drug conjugate | Ab-II |
|---|---|
| $EC_{50}$(nM) | 0.269 |

**Example 6: Confirmation of *in vitro* Anticancer Effect of anti-BCAM Antibody-Drug Conjugate Ab-II**

An *in vitro* experiment was conducted to confirm the anticancer effect of the Ab-II antibody-drug conjugate produced in Example 4 on human cell lines that express high levels of BCAM.

5 x $10^3$ cells of SK-BR-3 (human breast cancer cell line, KCLB), OVCAR3 (human ovarian cancer cell line, KCLB), and DU145 (human prostate cancer cell line, KCLB) were seeded into a 96-well plate (3595, Corning, USA). The next day, after confirming cell attachment to the plate, the existing cell culture was removed. Ab-II, produced in Example 4, was serially diluted in cell culture medium from 1 μM in a 5-fold dilution series across 8 points (down to 12.8 pM), and 200 μL was added to each well. On the day of drug treatment, the luminescence of the negative control (0% control, untreated with the drug) was measured. The culture medium was removed, 100 μL of CellTiter-Glo® (Promega, USA) solution was added to each well, and after reacting for 10 minutes at room temperature, the luminescence was measured with a microplate reader (Synergy $H_1$, Biotek, USA). The experimental group treated with the drug and the positive control group (100% control, untreated with the drug) were reacted for 72 hours in a 37°C $CO_2$ incubator. Afterward, the culture medium was removed, 100 μL of CellTiter-Glo® solution was added to each well, and after reacting for 10 minutes at room temperature, the luminescence was measured with the microplate reader. The % cell viability for Ab-II was calculated by substituting into the formula: [(Ab-II treated group) - (0% control group)] / [(100% control group) - (0% control group)]. The $GI_{50}$ (half-maximal growth inhibition concentration) value of Ab-II for each cell line was calculated using GraphPad Prism (GraphPad software Inc., USA) based on the % cell viability. The results are shown in FIG. 7 and Table 8.

From this, it was confirmed that the Ab-II antibody-drug conjugate of the present invention has excellent inhibitory ability against human cancer cell lines that express high levels of BCAM.

**[TABLE 8]**

| Antibody-drug conjugate | SK-BR-3 | OVCAR3 | DU145 |
|---|---|---|---|
| $GI_{50}$(nM) | 0.21 | 0.35 | 17 |

**[Sequence Listing]**

The sequence listing electronic file is attached.

## Claims

1. An anti-BCAM antibody or an antigen-binding fragment thereof that binds to a basal cell adhesion molecule (BCAM) protein, wherein the anti-BCAM antibody comprises: a heavy chain CDR1 comprising the amino acid sequence of

SEQ ID NO: 3; a heavy chain CDR2 comprising the amino acid sequence of SEQ ID NO: 4; a heavy chain CDR3 comprising the amino acid sequence of SEQ ID NO: 5; a light chain CDR1 comprising the amino acid sequence of SEQ ID NO: 6; a light chain CDR2 comprising the amino acid sequence of SEQ ID NO: 7; and a light chain CDR3 comprising the amino acid sequence of SEQ ID NO: 8.

**2.** The anti-BCAM antibody or antigen-binding fragment thereof of claim 1, comprising a heavy chain variable region consisting of the amino acid sequence of SEQ ID NO: 1 and a light chain variable region consisting of the amino acid sequence of SEQ ID NO: 2.

**3.** The anti-BCAM antibody or antigen-binding fragment thereof of claim 1 or 2, wherein the antibody is of an IgG isotype.

**4.** The anti-BCAM antibody or antigen-binding fragment thereof of any one of claims 1 to 3, wherein the antibody is a multi-specific antibody.

**5.** The anti-BCAM antibody or antigen-binding fragment thereof of any one of claims 1 to 4, wherein the antibody is a humanized antibody, a chimeric antibody, a CDR-grafted antibody, or a recombinant human antibody.

**6.** The anti-BCAM antibody or antigen-binding fragment thereof of any one of claims 1 to 5, wherein the antigen-binding fragment is Fab, Fab', Fab'-SH, Fv, a single-chain antibody scFv, an $F(ab')_2$ fragment, a light chain variable region (VL), a heavy chain variable region (VH), a diabody, a triabody, a tetrabody, a minibody, IgG delta $CH_2$, scFv-Fc, $(scFv)_2$-Fc, a Fynomer, a dual-affinity re-targeting (DART) protein, an anticalin, an $FN_3$ monobody, a DARPin, an Affibody, an Affilin, an Affimer, an Affitin, an Alphabody, an Avimer, Im7, VLR, VNAR, a Trimab, a CrossMab, a TRIDENT, a nanobody, a binanobody, or a di-sdFv.

**7.** The anti-BCAM antibody or antigen-binding fragment thereof of any one of claims 1 to 6, wherein it binds to human BCAM protein with a binding dissociation equilibrium constant ($K_D$) of 1 x $10^{-6}$ M or less.

**8.** An antibody-drug conjugate comprising the anti-BCAM antibody or antigen-binding fragment thereof of any one of claims 1 to 7.

**9.** A pharmaceutical composition for the prevention, amelioration, or treatment of a disease associated with BCAM function or expression, comprising:
(i) the anti-BCAM antibody or antigen-binding fragment thereof of any one of claims 1 to 7; or (ii) the antibody-drug conjugate of claim 8; and a pharmaceutically acceptable carrier.

**10.** The pharmaceutical composition of claim 9, wherein the disease associated with BCAM function or expression is cancer.

**11.** The pharmaceutical composition of claim 9 or 10, wherein the pharmaceutical composition is for use in combination with an additional anticancer agent.

**12.** The pharmaceutical composition of claim 11, for the prevention, amelioration, or treatment of a disease associated with BCAM function or expression, wherein the additional anticancer agent is administered simultaneously with the antibody or antigen-binding fragment thereof or the antibody-drug conjugate in a single formulation, or is administered simultaneously or sequentially in separate formulations.

**13.** A nucleic acid encoding the antibody or antigen-binding fragment thereof of any one of claims 1 to 7.

**14.** A recombinant expression vector comprising the nucleic acid of claim 13.

**15.** An isolated host cell for recombinantly producing the anti-BCAM antibody or antigen-binding fragment thereof of any one of claims 1 to 7.

**16.** A method for producing an antibody or an antigen-binding fragment thereof, the method comprising: culturing the host cell of claim 15 under conditions that allow for the production of the antibody or antigen-binding fragment thereof, and isolating the antibody or antigen-binding fragment thereof from the host cell or culture medium.

**17.** The anti-BCAM antibody or antigen-binding fragment thereof of any one of claims 1 to 7, for use in the detection,

diagnosis, and/or treatment of a disease associated with BCAM function or expression.

[Figure 1]

| Region | Sequence |
|---|---|
| Ab-I VH | EVQLVESGGGLVKPGGSLRLSCAASGFTFSSYSMNWVRQAPGKGLEWVS SISSSSSYIYYADSVKGRFTISRDNAKNSLYLQMNSLRAEDTAVYYC ARIIGGWLGDYWGQGTLVTVSS |
| Region | Sequence |
| Ab-I VL | NIQMTQSPSSVSASVGDRVTITCRASQGISSWLAWYQQKPGKAPKLLIY AASSLRSGVPSRFSGSGSGTDFTLTISSLQPEDFATYYCQQANSFPPPT FGGGTKVEIK |

[Figure 2]

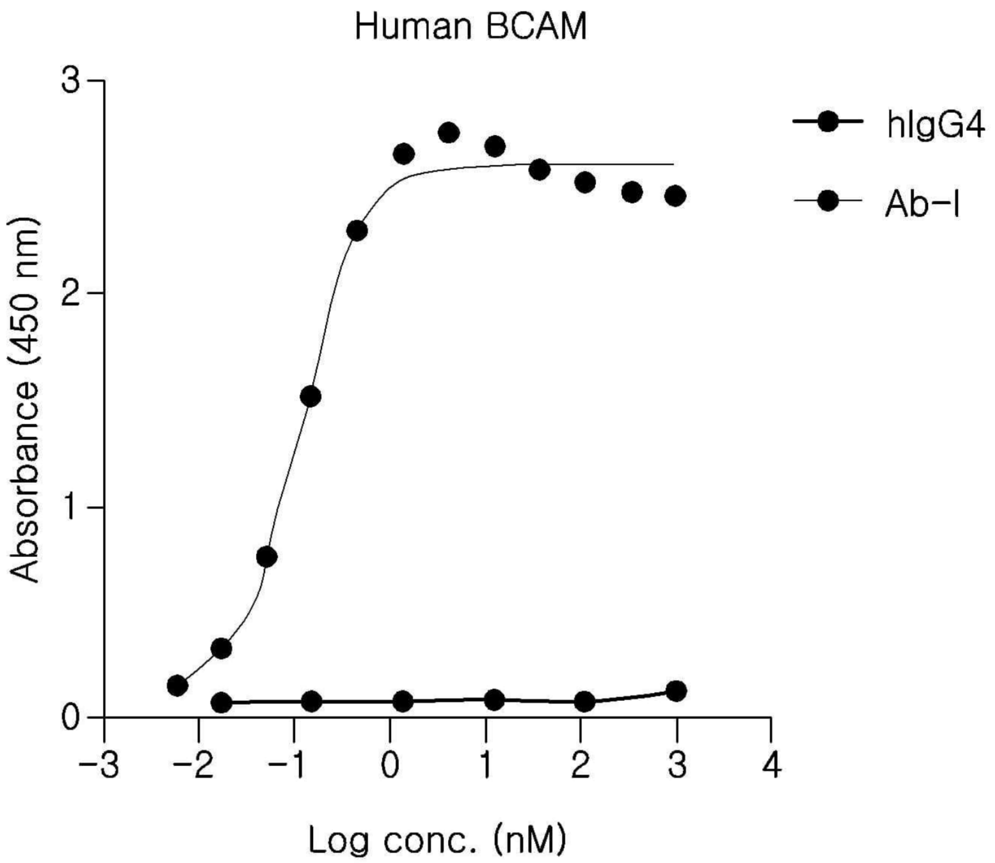

[Figure 3]

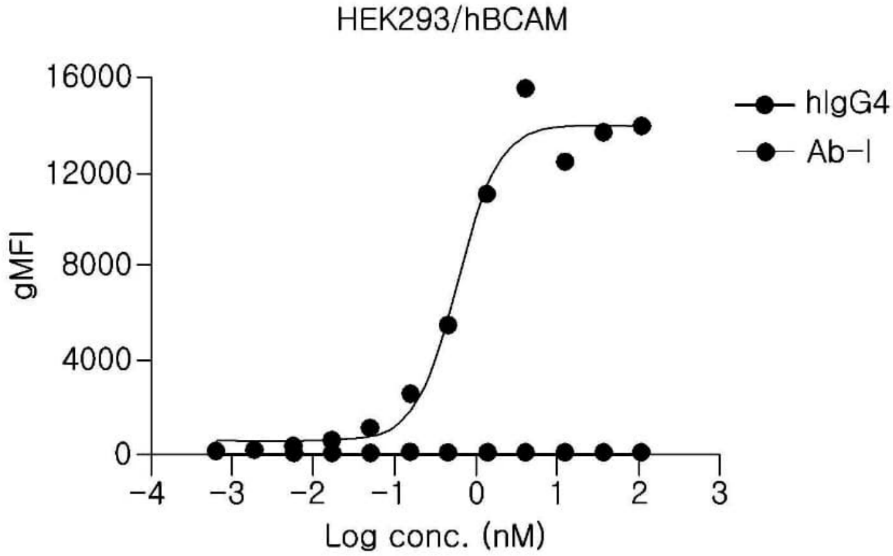


[Figure 4]

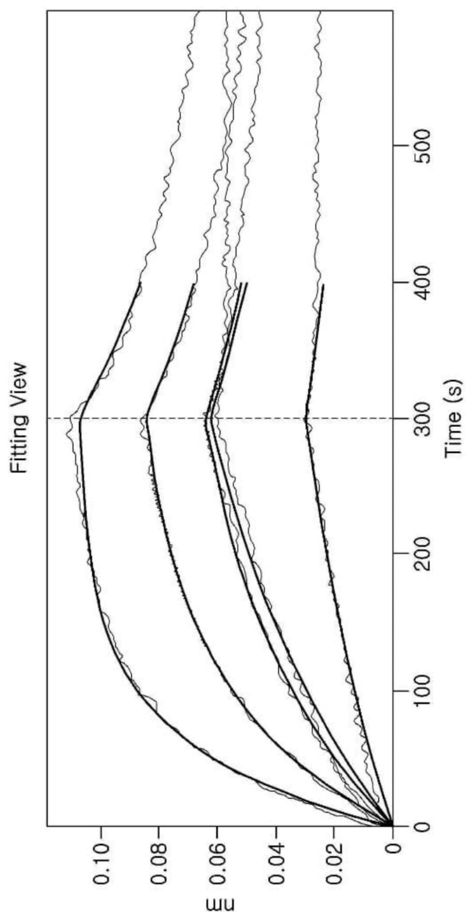

[Figure 5]

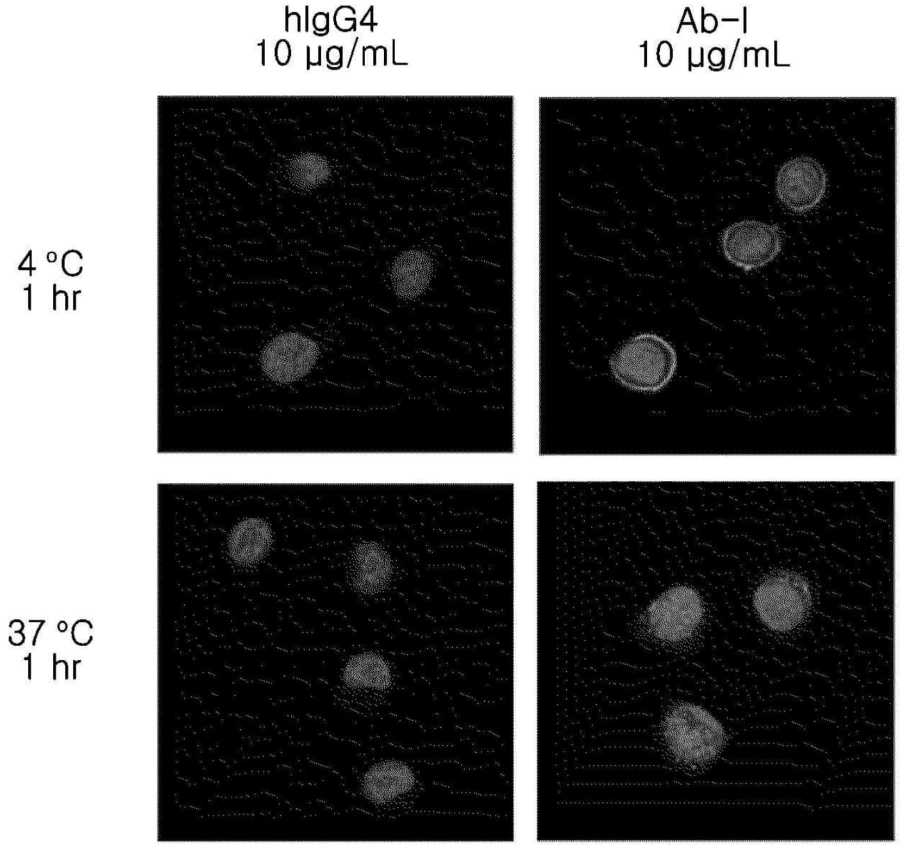


[Figure 6]

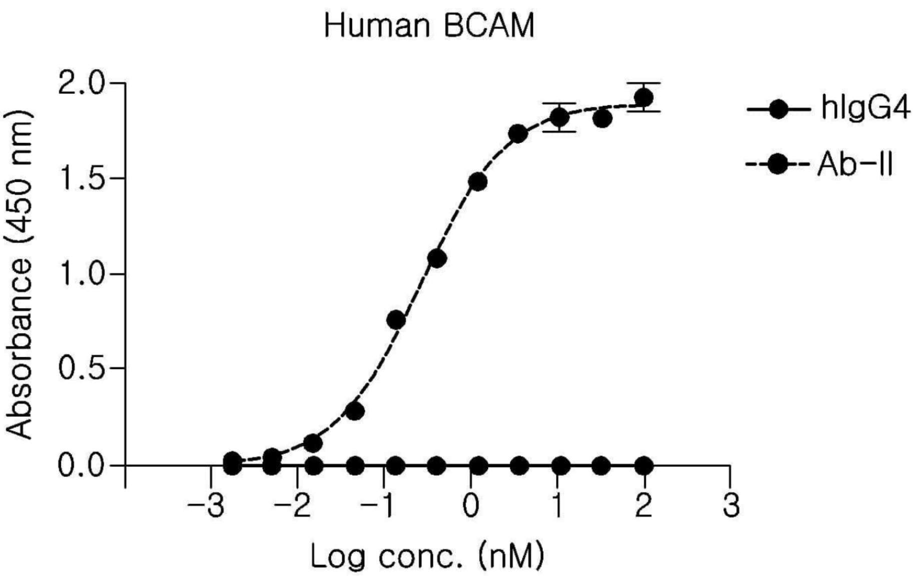

[Figure 7]

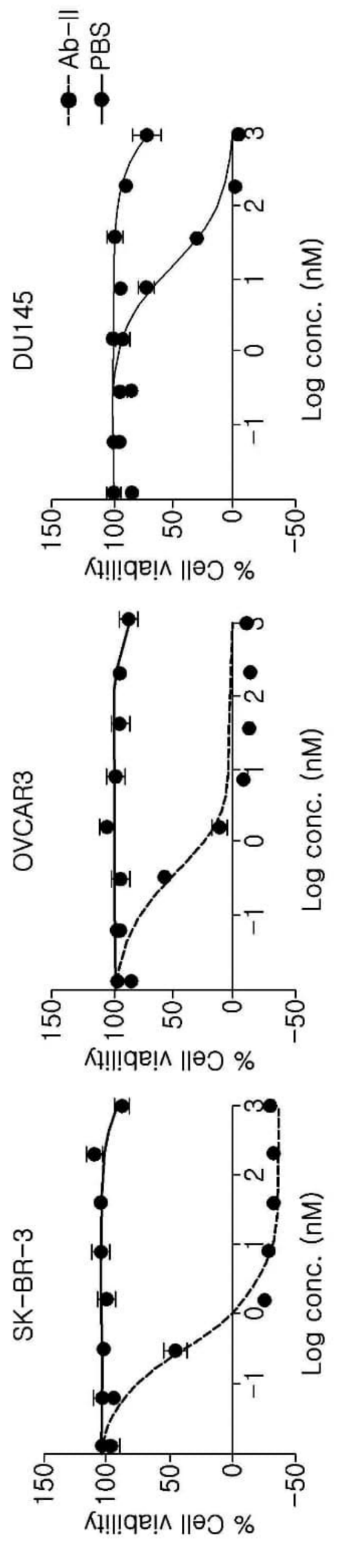

## INTERNATIONAL SEARCH REPORT

International application No.
**PCT/KR2024/004194**

**A. CLASSIFICATION OF SUBJECT MATTER**

**C07K 16/28**(2006.01)i; **A61K 47/68**(2017.01)i; **A61P 35/00**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

C07K 16/28(2006.01); A61K 39/00(2006.01); A61K 39/395(2006.01); C12N 15/09(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: BCAM(Basal Cell Adhesion Molecule), 항체(antibody), CDR, 항체-약물 컨쥬게이트(antibody-drug conjugate), 치료(treatment)

**C. DOCUMENTS CONSIDERED TO BE RELEVANT**

| Category* | Citation of document, with indication, where appropriate, of the relevant passages | Relevant to claim No. |
|---|---|---|
| A | YU, Hyunkyung et al. Abstract 1760: the antibody-drug conjugate GENA-111 conjugated to auristatin F shows therapeutic potency in BCAM positive epithelial cancer. Cancer Research. 15 June 2022, Volume 82, Issue 12_Supplement, Abstract. Retrieved from <https://doi.org/10.1158/1538-7445.AM2022-1760>. See abstract. | 1-3 |
| A | KR 10-2022-0157686 A (GENOME＆COMPANY, INC.) 29 November 2022 (2022-11-29) See entire document. | 1-3 |
| A | JP 2017-095417 A (TOKYO UNIVERSITY OF PHARMACY & LIFE SCIENCES et al.) 01 June 2017 (2017-06-01) See entire document. | 1-3 |
| DA | KIKKAWA, Yamato et al. Internalization of CD239 highly expressed in breast cancer cells: a potential antigen for antibody-drug conjugates. Scientific Reports. 26 April 2018 (online publication date), vol. 8, article no. 6612, pp. 1-11. See entire document. | 1-3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 June 2024** | **01 July 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No. **PCT/KR2024/004194**

**C. DOCUMENTS CONSIDERED TO BE RELEVANT**

| Category* | Citation of document, with indication, where appropriate, of the relevant passages | Relevant to claim No. |
|---|---|---|
| A | SCHRÖFELBAUER, Bärbel et al. Discovery of antibodies and cognate surface targets for ovarian cancer by surface profiling. PNAS. 27 December 2022, vol. 120, no. 1, e2206751120, pp. 1-11. See entire document. | 1-3 |

Form PCT/ISA/210 (second sheet) (July 2022)

**INTERNATIONAL SEARCH REPORT**

International application No.
**PCT/KR2024/004194**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT

International application No.
**PCT/KR2024/004194**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **10,12,14,16**
because they relate to parts of the international application that do not comply with the prescribed requirements to such an extent that no meaningful international search can be carried out, specifically:

Claims 10, 12, 14 and 16 refer to claims violating the manner of referring to dependent claims (PCT Rule 6.4(a)), and thus are unclear.

3. ☑ Claims Nos.: **4-9,11,13,15,17**
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/004194**

| Patent document cited in search report | Publication date (day/month/year) | Patent family member(s) | Publication date (day/month/year) |
|---|---|---|---|
| KR 10-2022-0157686 A | 29 November 2022 | CN 117396507 A | 12 January 2024 |
| | | EP 4342914 A1 | 27 March 2024 |
| | | WO 2022-244908 A1 | 24 November 2022 |
| JP 2017-095417 A | 01 June 2017 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230044305
- WO 2008022759 A
- US 20100305304 A


### Non-patent literature cited in the description


- **YAMATO KIKKAWA et al.** *Scientific Report*, 26 April 2018, vol. 8 (1), 6612
- **MORRISON**. *Science*, 1985, vol. 229, 1202
- **D GRABULOVSKI et al.** *Journal of Biological Chemistry*, 2007, vol. 282 (5), 3196-3204
- **S. JOHNSON et al.** *Journal of Molecular Biology*, 2010, vol. 399 (3), 436-449
- **A. SKERRA**. *Current Opinion in Biotechnology*, 2007, vol. 18 (4), 295-304
- **KOIDE et al.** *Journal of Molecular Biology*, 1998, vol. 284 (4), 1141-1151
- Engineering of Affibody Molecules for Therapy and Diagnostics.. **FELDWISCH, J.** ; **TOLMACHEV, V.** Therapeutic Proteins. Methods in Molecular Biology. Humana Press, 2012, vol. 899
- **B MOURATOU et al.** *Biomolecules.*, 30 January 2015, vol. 5 (1), 60-75
- **J DESMET et al.** *Nature Communications*, 2014, vol. 5
- **UH WEIDLE et al.** *Cancer Genomics Proteomics.*, July 2013, vol. 10 (4), 155-68
- **BOEHM et al.** *Annual Review of Immunology*, vol. 30, 203-220
- **LEE et al.** *Molecular Therapy*, 2014, vol. 22 (7), 1254-1265
- **JAYASENA, S.D.** *Clin. Chem.*, 1999, vol. 45, 1628-50
- **FELLOUSE, F.A. et al.** *J. Mol. Biol.*, 2007, vol. 373 (4), 924-40
- **F.R.M. LATINI et al.** *Blood Cells, Molecules and Diseases*, 2013, vol. 50, 161-165